# EUROPÄISCHE PATENTSCHRIFT

(11) **EP 3 733 238 B1**
(45) Veröffentlichungstag und Bekanntmachung des Hinweises auf die Patenterteilung: **06.04.2022**
(21) Anmeldenummer: 19171963.2
(22) Anmeldetag: 30.04.2019
(51) Int. Cl.: A61N 1/05

(54) **FLEXIBLE ELEKTRODE AUS EINEM METALLISCHEN GRUNDMATERIAL**
FLEXIBLE ELECTRODE MADE OF A METALLIC SUBSTRATE
ÉLECTRODE FLEXIBLE D'UN MATÉRIAU DE BASE MÉTALLIQUE

(43) Veröffentlichungstag der Anmeldung: 04.11.2020
(73) Patentinhaber: Friedrich-Alexander-Universität Erlangen-Nürnberg, 91054 Erlangen (DE); Matthias Karl GmbH, 93449 Waldmünchen (DE)
(72) Erfinder: ROSIWAL, Stefan, 96049 Bamberg (DE); KARL, Matthias, 93449 Waldmünchen (DE); BURKOVSKI, Andreas, 91052 Erlangen (DE); GÖLTZ, Maximilian, 91096 Möhrendorf (DE)
(74) Vertreter: Dr. Gassner & Partner mbB

(56) Entgegenhaltungen:
- EP-A2- 0 994 074
- WO-A2-02/45589
- CN-U- 207 646 292
- MARÍA ALCAIDE ET AL: "Boron-Doped Nanocrystalline Diamond Electrodes for Neural Interfaces: In vivo Biocompatibility Evaluation", FRONTIERS IN NEUROSCIENCE, Bd. 10, 8. März 2016 (2016-03-08), XP055543134, DOI: 10.3389/fnins.2016.00087

## Beschreibung

Die Erfindung betrifft eine flexible Elektrode aus einem metallischen Grundmaterial mit einer Beschichtung aus polykristallinem dotiertem elektrisch leitfähigem Diamant.

Aus Ochiai, T. et al., ChemPhysChem 2013, 14, Seiten 2094 bis 2096 ist eine Bor-dotierte Diamantmikroelektrode zur Zahnbehandlung bekannt. Das Grundmaterial der Elektrode besteht aus Wolfram, welches mit Bor-dotiertem Diamant unter Verwendung eines mikrowellenplasmaunterstützten chemischen Dampfabscheidungssystems beschichtet worden ist. Die Elektrode hatte einen Durchmesser von 500 µm und eine polykristalline Diamantkorngröße von etwa 2 µm. Die Elektrode wurde mit einem Streifen einer Nafion^{®}-Ionenaustauschmembran spiralförmig umwickelt. Um die lonenaustauschmembran wurde ein Platindraht gewickelt. Im Einsatz der Elektrode wurde die Bor-dotierte Diamant-Elektrode als Anode und der Platindraht als Kathode geschaltet. Dabei wurden an der Anode Ozon, OH-Radikale und oxidative Zwischenprodukte gebildet. Diese Spezies weisen einen desinfizierenden Effekt auf.

Aus der WO 2016/017694 ist ein elektrisch leitfähiges Beschichtungsmaterial bekannt, welches Bor-dotiertes Diamantpulver und eine lonen-Austausch-Harz-Dispersion enthält. Weiterhin wird eine mit diesem Beschichtungsmaterial beschichtete nadelförmige oder scheibenförmige Elektrode und deren Anwendung zur Behandlung von Karies und Parodontitis sowie zur Behandlung des Wurzelkanals offenbart. Als Vorteil der Elektrode wird beschrieben, dass die Beschichtung nicht leicht von der Elektrode abblättert. Zur Anwendung der Elektrode wird beschrieben, dass eine Elektrolyseeinheit verwendet wird, welche neben der genannten Elektrode und einer Gegenelektrode eine zwischen der Elektrode und der Gegenelektrode angeordnete lonenaustauschmembran aufweist. Aus der EP 0 994 074 A2 ist eine weitere Elektrode bekannt.

Aus Ping J. et al., Nat Commun. 2015;6:8050, August 2015 ist ein durch elektrische Aktivierung aushärtender Kunststoff bekannt.

Das Problem bei der Behandlung eines Wurzelkanals besteht darin, dass dieser üblicherweise gekrümmt verläuft und darüber hinaus häufig in mehrere Kanäle unterteilt ist. Weiterhin weist die umgebende Zahnhartsubstanz eine hohe Porosität auf. Um eine möglichst gründliche Desinfektion eines Wurzelkanals mit einer mit leitfähigem Diamant beschichteten Elektrode zu erreichen, sollte diese möglichst tief in die Zahnwurzel eingeführt werden. Dazu ist es erforderlich, dass die Elektrode flexibel ist. Eine direkt auf der Elektrode aufgebrachte Diamantbeschichtung ist jedoch grundsätzlich spröde und blättert beim Verbiegen der Elektrode ab. Aufgabe der vorliegenden Erfindung ist es daher, eine mit leitfähigem Diamant beschichtete flexible Elektrode anzugeben, welche beispielsweise für eine Wurzelkanalbehandlung geeignet ist.

Die Aufgabe wird durch die Merkmale des Patentanspruchs 1 gelöst. Zweckmäßige Ausgestaltungen ergeben sich aus den Merkmalen der Patentansprüche 2 bis 15.

Die erfindungsgemäße flexible Elektrode weist ein metallisches Grundmaterial mit einer Beschichtung aus polykristallinem dotiertem elektrisch leitfähigem Diamant und einer Zwischenschicht zwischen dem Grundmaterial und der Beschichtung auf. Das Grundmaterial ist dabei nadelförmig, drahtförmig oder bandförmig ausgebildet. Es kann einen Durchmesser bzw. maximale Länge der Diagonale durch die Querschnittsfläche zwischen 100 und 1000 µm, insbesondere zwischen 200 und 800 µm, insbesondere zwischen 300 und 600 µm, aufweisen.

In dem Grundmaterial ist um eine Längsachse des Grundmaterials herum mindestens eine umlaufende, insbesondere kreisförmig oder spiralförmig verlaufende, Auskerbung enthalten. Die Auskerbung mit der Zwischenschicht und der Beschichtung, d. h. die beschichtete Auskerbung, kann dabei tiefer sein als die Summe der maximalen Stärke der Beschichtung und der maximalen Stärke der Zwischenschicht. Die Tiefe der beschichteten Auskerbung wird dabei gemessen zwischen dem höchsten die beschichtete Auskerbung direkt umgebenden Punkt und dem tiefsten Punkt der beschichteten Auskerbung. Der höchste die beschichtete Auskerbung direkt umgebende Punkt kann auch in einem parallel zur Längsachse der Elektrode verlaufenden hier nicht dargestellten Bereich liegen. In diesem Fall gibt es dann eine Mehrzahl gleich hoch gelegener, höchster die beschichtete Auskerbung direkt umgebender Punkte.

Die Tiefe der beschichteten Auskerbung und die maximale Stärke der Beschichtung und der Zwischenschicht lassen sich beispielsweise elektronenmikroskopisch bestimmen. In einer elektronenmikroskopischen Aufnahme eines durch die Längsachse der Elektrode verlaufenden Schnitts durch die Elektrode können dazu parallel zur Längsachse der Elektrode Hilfslinien durch den höchsten die beschichtete Auskerbung direkt umgebenden Punkt und den tiefsten Punkt der beschichteten Auskerbung gezogen und der Abstand zwischen diesen beiden Linien bestimmt werden. Dieser Abstand kann dann als Tiefe der beschichteten Auskerbung erachtet werden. Wenn auf diese Weise die mittels einer bestimmten Technik unter bestimmten Bedingungen erzeugte beschichteten Auskerbung eine gewünschte Tiefe hat, kann davon ausgegangen werden, dass weiteres unter gleichen Bedingungen gleich bearbeitetes Grundmaterial in ebensolchen Auskerbungen resultiert. Alternativ kann die Tiefe einer Auskerbung auch zerstörungsfrei mittels eines Rasterelektronenmikroskops, mittels Röntgenstrahlung, durch Oberflächenabtastung mittels eines Elektronenstrahls oder sogar Lichtmikroskopisch, z. B. mittels eines konfokalen Lasermikroskops, bestimmt werden. Die Schichtdicke der Zwischenschicht und der Beschichtung kann durch die Wahl der Parameter beim Beschichten so gut vorhergesagt werden, dass keine Messung erforderlich ist, um die Elektrode so herzustellen, dass diese eine beschichtete Auskerbung aufweist, die tiefer ist als die Summe der maximalen Stärke der Beschichtung und der maximalen Stärke der Zwischenschicht. Die Auswirkungen der Parameter und deren Änderung auf die Schichtdicke der Beschichtung und der Zwischenschicht beim Beschichten sind in der Technik wohlbekannt.

Die Auskerbung kann in dem Grundmaterial beispielsweise durch Laserabtragung mittels eines Kurzpulslasers, wie einem Neodym YAG-Laser, und einer nachträglichen Entfernung dabei gebildeter Oxide mittels Partikelstrahlen oder einer Säure gebildet werden. Auch eine mechanische Strukturierung der Elektrodenoberfläche mittels eines Schleif- oder Pressprozesses oder mittels Partikelstrahlen ist möglich. Bei den für das Partikelstrahlen verwendeten Partikeln kann es sich beispielsweise um Siliziumkarbid-Partikel handeln. Diese können beispielsweise einen Durchmesser im Bereich von 30 µm bis 300 µm oder weniger aufweisen. Die Partikel können beispielsweise mit einem Druck im Bereich von 3 bar bis 5 bar oder mehr auf die Elektrode gestrahlt werden.

Die Wirkung der Auskerbung besteht darin, dass dadurch Biegestellen in dem Grundmaterial vorgegeben werden. Im Falle einer oder mehrerer umlaufenden kreisförmigen oder im Falle eines bandförmigen Grundmaterials rechteckförmigen Auskerbung(en) wird eine Segmentierung der Elektrode erreicht. Das Besondere im Zusammenhang mit der Beschichtung besteht darin, dass ein Verbiegen der Elektrode Im Bereich der Auskerbung(en) stattfindet und dort in der Beschichtung lediglich kleine Risse tief in der Auskerbung entstehen, die die Funktionalität der Elektrode nicht oder allenfalls gering beeinflussen. Bei elektrochemischen Messungen wurde festgestellt, dass die Beschichtung auch nach einem Verbiegen der Elektrode auf mehr als 90 % ihrer Gesamtfläche intakt ist.

Dadurch wird eine bisher unerreichte Flexibilität der Elektrode erreicht, welche es ermöglicht, diese tief in einen gekrümmt verlaufenden Wurzelkanal eines Zahns einzuführen und dort OH-Radikale und andere desinfizierende Spezies zu erzeugen, um eine bisher unerreicht gründliche Desinfektion des Wurzelkanals zu ermöglichen.

Der Diamant kann mit Bor oder Phosphor dotiert sein. Bei dem Grundmaterial kann es sich um Titan, Niob, Tantal, Eisen oder eine diese Metalle enthaltende Legierung handeln. Bei der Legierung kann es sich um Ti-6Al-4V, Ti-6Al-7Nb oder einer sonstigen Legierung, die neben Titan Aluminium und/oder Niob und/oder Eisen und/oder Molybdän enthält, handeln. Das Grundmaterial kann auch aus Stahl bestehen.

Die Zwischenschicht kann aus einem Metallkarbid, einem Metallnitrid, einem Metallborid oder einer mindestens zwei der genannten Karbide enthaltenden Mischverbindung bestehen. Die Zwischenschicht kann eine Schichtdicke von höchstens 10 µm, insbesondere höchstens 5 µm, aufweisen. Die Zwischenschicht kann weiterhin eine Schichtdicke von mindestens 50 nm, insbesondere mindestens 100 nm, aufweisen. Sowohl die Schichtdicke der Zwischenschicht als auch die Schichtdicke des Diamanten ist durch die Beschichtungsbedingungen beeinflussbar. Dies ist beispielsweise aus der Dissertation Neuerer, K. "Beeinflussung der Titankarbid-Schichtdicke bei der HFCVD-Diamantbeschichtung von Titan durch Oberflächenvorbehandlungen und Variation der Beschichtungsparameter" der Technischen Fakultät der Universität Erlangen-Nürnberg, 2013 bekannt. Die Elektrode kann durch Heißdraht-aktivierte chemische Gasphasenabscheidung (hot filament chemical vapor deposition = HFCVD) oder durch mikrowellenplasmaunterstützte chemische Gasphasenabscheidung (microwave plasma-assisted chemical vapor deposition = MPCVD) oder durch eine sonstige chemische Gasphasenabscheidung (chemical vapor deposition = CVD) an der Oberfläche der Elektrode in einer eine borhaltige Verbindung enthaltenden Gasphase mit Diamant beschichtet werden.

Bei einer Ausgestaltung weist die Beschichtung mit Diamant eine Schichtdicke von höchstens 3 µm, insbesondere weniger als 2 µm, insbesondere weniger als 1 µm, und von mindestens 100 nm auf. Durch die geringe Schichtdicke wird eine zusätzliche Flexibilität der Elektrode bei dennoch ausreichender Dichtheit der Beschichtung erreicht.

Die Erfindung betrifft weiterhin eine erfindungsgemäße Elektrode zur Verwendung bei einer antimikrobiellen Behandlung eines mikrobiell infizierten Endodonts oder Parodonts eines Zahnes oder eines mikrobiell infizierten periimplantären Gewebes eines Säugetiers oder Menschen. Bei dem Endodont kann es sich insbesondere um einen mikrobiell infizierten Wurzelkanal und bei dem Parodont um ein mikrobiell infiziertes Zahnfleisch, insbesondere im Bereich der Zahnfleischtasche, handeln. Bei der mikrobiellen Infektion kann es sich um eine Infektion durch Prokaryoten, insbesondere eine bakterielle Infektion, oder durch Eukaryoten, insbesondere eine Mykose, handeln.

Bei der Behandlung eines Wurzelkanals wird die Elektrode tief in den Wurzelkanal eingeführt. Eine Gegenelektrode kann außerhalb des Wurzelkanals angeordnet sein. Die erfindungsgemäße Elektrode wird bei der antimikrobiellen Behandlung als Anode geschaltet, so dass sich daran Hydroxidradikale, Ozon und, insbesondere beim Vorhandensein von Chlorid-Ionen, weitere antimikrobiell wirksame Spezies, wie z. B. Chlorat-Ionen (ClO₃⁻), bilden. Bei der Behandlung des Wurzelkanals kann dieser mit einem Elektrolyten, beispielsweise einer physiologischen Kochsalzlösung, gefüllt werden, wenn sich darin keine Körperflüssigkeit befindet, die generell als natürlicher Elektrolyt dienen kann.

Auch bei der Behandlung der mikrobiell infizierten Zahnfleischtasche kann die erfindungsgemäße Elektrode tief in die Zahnfleischtasche eingeführt werden und die Gegenelektrode außerhalb angeordnet sein. Gegebenenfalls kann die Zahnfleischtasche mit physiologischer Kochsalzlösung gespült werden, um einen Elektrolyten bereit zu stellen. Als natürlicher Elektrolyt ist jedoch auch Speichel geeignet.

Bei einer Ausgestaltung der erfindungsgemäßen Elektrode bildet diese mit einer weiteren Elektrode eine Doppelelektrode. Dabei ist die weitere Elektrode ebenfalls nadelförmig, drahtförmig oder bandförmig ausgebildet. Die weitere Elektrode kann beispielsweise aus korrosionsbeständigem Stahl bestehen. Eine Beschichtung dieser Elektrode ist nicht erforderlich. Die Elektrode und die weitere Elektrode können in einem Bereich durch mindestens ein Verbindungsmittel oder ein Verbindungsmittel und ein Abstandshaltemittel so miteinander verbunden sein, dass dadurch ein direkter oder indirekter elektrischer Kontakt zwischen der Elektrode und der weiteren Elektrode ausgeschlossen ist. Das Verbindungsmittel und das Abstandshaltemittel sind daher elektrisch nicht leitfähig. Weiterhin können die Elektrode und die weitere Elektrode durch das Verbindungsmittel oder das Verbindungsmittel und das Abstandshaltemittel so miteinander verbunden sein, dass in mindestens 80 % der Länge des Bereichs zwischen der Elektrode und der weiteren Elektrode ein freier, bei der Behandlung von einem Elektrolyten einzunehmender Raum gewährleistet ist, wobei ein Abstand zwischen der Elektrode und der weiteren Elektrode innerhalb dieses Raums zwischen 40 µm und 300 µm beträgt. Der Bereich wird dabei durch die jeweils äußersten Stellen beschränkt, an welchen die Elektrode und die weitere Elektrode durch das Verbindungsmittel miteinander verbunden sind. Durch den geringen Abstand zwischen der Elektrode und der weiteren Elektrode und dadurch, dass dieser Raum frei ist und dort insbesondere keine lonenaustauschmembran vorgesehen ist, wird eine sehr effiziente Erzeugung von OH-Radikalen bei verhältnismäßig geringem Stromfluss erreicht. Die Effizienz ist höher als bei einer von der Gegenelektrode durch eine Ionenaustauschmembran getrennten diamantbeschichteten Elektrode. Die Elektrode muss dabei auf ihrer Oberfläche zumindest innerhalb des Bereichs eine Beschichtung mit polykristallinem dotiertem elektrisch leitfähigem Diamant aufweisen.

Auch die weitere Elektrode kann sehr dünn ausgebildet sein, so dass auch die gebildete Doppelelektrode insgesamt nur einen geringen maximalen Durchmesser aufweist, so dass damit gut mikrobiell infizierte Zahnfleischtaschen und gegebenenfalls ausreichend weite Wurzelkanäle behandelt werden können. Bei der Behandlung kann die Elektrode als Anode und die weitere Elektrode als Kathode geschaltet sein. Für eine zahnärztliche oder kieferchirurgische Behandlung kann die Elektrode und gegebenenfalls auch die weitere Elektrode von einem, insbesondere zahnärztlichen oder kieferchirurgischen Instrumentenhalter gehalten und mit Strom versorgt werden.

Bei einer Ausgestaltung der erfindungsgemäßen Elektrode, die mit der weiteren Elektrode eine Doppelelektrode bildet, besteht die weitere Elektrode aus demselben Grundmaterial wie die Elektrode und ist wie die Elektrode aufgebaut. In diesem Fall ist die weitere Elektrode ebenfalls mit Diamant beschichtet. Dadurch kann eine flexible Doppelelektrode bereit gestellt werden, die bei der Behandlung umgepolt oder mit Wechselstrom betrieben werden kann. Dadurch kann die Bildung von Ablagerungen an der Anode, welche die Effizienz der Anode reduzieren können und unter Umständen sogar einen Kurzschluss zwischen beiden Elektroden bewirken können, vermieden werden. Dazu werden bei der Behandlung die Elektrode und die weitere Elektrode jeweils abwechselnd als Anode und die jeweils andere Elektrode als Kathode geschaltet. Weiterhin wird durch die Umpolung und die damit einhergehende Gasbildung an der zuvor als Anode geschalteten Kathode die Verteilung der gebildeten OH-Radikale und anderer antimikrobiell wirksamer Spezies verbessert.

Bei einer Ausgestaltung der Elektrode, die mit der weiteren Elektrode eine Doppelelektrode bildet, beträgt der Abstand zwischen der Elektrode und der weiteren Elektrode innerhalb des Raums zwischen 40 und 200 µm, insbesondere zwischen 40 und 110 µm.

Je geringer der Abstand ist, desto geringer sind die zur Erzielung der Wirkung erforderlichen Stromstärken und desto effizienter werden OH-Radikale gebildet.

Bei dem Verbindungsmittel kann es sich um einen elektrisch isolierenden Klebstoff, insbesondere ein Epoxidharz, oder einen nicht leitenden Diamanten handeln. Bei dem Abstandshaltemittel kann es sich um Teflon, einen Kunststofffaden oder einen elektrisch isolierend beschichteten Draht handeln. Bei dem Kunststofffaden kann es sich um einen, insbesondere teflonbeschichteten, Polyethylenfaden, insbesondere aus Polyethylen mit ultrahohem Molekulargewicht, handeln.

Eine mittels elektrisch nicht leitendem Diamant als Verbindungsmittel verbundene Doppelelektrode kann beispielsweise folgendermaßen hergestellt werden:
Die erfindungsgemäße mit Diamant beschichtete flexible Elektrode wird an sämtlichen Stellen, an denen kein Verbindungsmittel gebildet werden soll, mit Kupfer beschichtet oder mit Kupferfolie umwickelt. Die Kupferschicht kann ca. 5-50 µm dick sein. Sie kann beispielsweise mittels Sputtern erzeugt werden. Beim Sputtern können Bereiche, die nicht beschichtet werden sollen mittels einer Maske oder durch Abdecken freigehalten werden. Für die Herstellung der Doppelelektrode wird die Beschichtung bzw. Abdeckung mit Kupfer jeweils alle 1 mm bis 5 mm für jeweils 0,1 mm bis 1 mm unterbrochen, so dass an diesen Stellen die Diamantschicht frei liegt.

Anschließend wird die weitere aus Metall bestehende Elektrode mittels Kupferfolie so an der flexiblen Elektrode befestigt, dass sich die kupferbedeckten und nicht kupferbedeckten Bereiche decken. Dadurch liegen die kupferfreien Bereiche der beiden Elektroden im Abstand von 5-50 µm genau gegenüber. Die weitere Elektrode kann aus Titan, Niob, Tantal oder Stahl mit einer TiNB-Zwischenschicht oder aus einem mit polykristallinem dotiertem elektrisch leitfähigem Diamant beschichtetem Metall bestehen. Es kann sich dabei auch um eine erfindungsgemäße flexible diamantbeschichtete Elektrode handeln. Falls die weitere Elektrode nicht diamantbeschichtet ist, wird diese, beispielsweise mittels HFCVD, MPCVD oder CVD mit Nanodiamant bekeimt. Dies kann vor oder nach der Befestigung an der flexiblen Elektrode erfolgen. Wichtig ist dabei, dass die nicht kupferbedeckten Bereiche bekeimt werden.

Die Bildung der Verbindung aus nicht leitendem Diamant erfolgt mittels CVD von Diamant ohne Bor. Dabei wachsen die gegenüberliegenden freiliegenden Bereiche an den Elektroden zusammen. Dadurch entstehen elektrisch nicht leitende, mechanisch feste Verbindungen zwischen den Elektroden. Die minimale Schichtdicke der nicht leitenden Diamantschicht entspricht dem halben Elektrodenabstand. Sie liegt etwa im Bereich von 5 µm bis 50 µm).

Zuletzt wird die Kupferfolie mechanisch entfernt. Durch Sputtern erzeugte Kupferbeschichtungen werden durch Ätzen mittels einer Säure entfernt. Dadurch werden die gegenüberliegenden, elektrisch aktiven Bereiche der Doppelelektrode freigelegt.

Die Behandlung des mikrobiell infizierten Endodonts kann nach der antimikrobiellen Behandlung ein zumindest teilweises Verfüllen eines im Zahn bestehenden oder durch eine weitere Behandlung, z. B. ein Aufbohren des Wurzelkanals, gebildeten Hohlraums mit einem durch elektrische Aktivierung im Hohlraum aushärtenden Kunststoff umfassen. Dabei erfolgt die elektrische Aktivierung mittels der Elektrode im Hohlraum.

Bei dem Hohlraum kann es sich also um einen aufgebohrten oder einen lediglich vom darin enthaltenen biologischen Material befreiten Wurzelkanal gegebenenfalls mit den in den Wurzelkanal mündenden Dentinkanälchen handeln.

Bei dem Kunststoff kann es sich beispielsweise um ein Polyamidoamin-g-diazirin handeln. Der Kunststoff ist beispielsweise aus Ping J. et al., Nat. Commun. 2015;6:8050, August 2015 bekannt. Zur elektrischen Aktivierung wird die erfindungsgemäße Elektrode mit einer Gegenelektrode verwendet. Da die Initiierung und das Fortschreiten der Vernetzung des Kunststoffs spannungsabhängig und zeitabhängig erfolgt, ist die Aushärtung durch die elektrische Aktivierung sehr gut steuerbar.

Die Behandlung kann auch ein zumindest teilweises Verfüllen des Hohlraums mit einem anorganischen Stoff, dessen Präzipitation aus einer Lösung im Hohlraum durch anodische Oxidation initiiert wird, umfassen. Dabei erfolgt die anodische Oxidation mittels der Elektrode. Eine Gegenelektrode kann dazu außerhalb des Zahns oder ebenfalls im Hohlraum, z. B. beim Einsatz der oben beschriebenen Doppelelektrode, angeordnet sein. Zur Präzipitation kann beispielsweise ein Phosphonat, wie z. B. ein Metalldiethylphosphonat, insbesondere Aluminiumdiethylphosphonat, durch anodische Oxidation mittels der erfindungsgemäßen Elektrode in ein unlösliches Orthophosphat überführt werden. Die Präzipitation kann durch eine chemische Reaktion verstärkt werden. Dazu kann das Orthophosphat z. B. mit einem Metallhalogenid, insbesondere einem Metallchlorid, insbesondere Eisen(III)-chlorid (FeCl₃), umgesetzt werden. Dadurch wird dann das entsprechende Metallphosphat, beispielsweise Eisen(III)-phosphat, präzipitiert.

Sowohl bei dem Verfüllen mit dem Kunststoff als auch mit dem anorganischen Stoff kann ein teilweises Verfüllen genügen. Dabei kann es genügen, wenn dadurch die Dentinkanälchen verschlossen werden, so dass daraus keine Mikroorganismen mehr in den Wurzelkanal eindringen können.

Nachfolgend wird die Erfindung anhand von Ausführungsbeispielen näher erläutert.

Es zeigen
- Fig. 1: eine schematische Darstellung einer erfindungsgemäßen Elektrode, die mit einer weiteren Elektrode eine Doppelelektrode bildet,
- Fig. 2: eine schematische Darstellung eines Zahns und eines zahnärztlichen In-strumentenhalters mit einer davon gehaltenen und mit Strom versorgten erfindungsgemäßen Elektrode als Bestandteil einer Doppelelektrode,
- Fig. 3: einen schematisch im Querschnitt dargestellten Versuchsaufbau zur Inak-tivierung von *Staphylococcus epidermidis* und *Bacillus subtilis* in einem aufgebohrten Wurzelkanal eines menschlichen Zahns,
- Fig. 4: ein Diagramm zur Darstellung der Abhängigkeit des Wachstums von *Staphylococcus epidermidis* von der Ladungsmenge und der Dauer der Behandlung des Wurzelkanals und
- Fig. 5: ein Diagramm zur Darstellung der Abhängigkeit des Wachstums von *Bacil-lus subtilis* von der Ladungsmenge und der Dauer der Behandlung des Wurzelkanals.

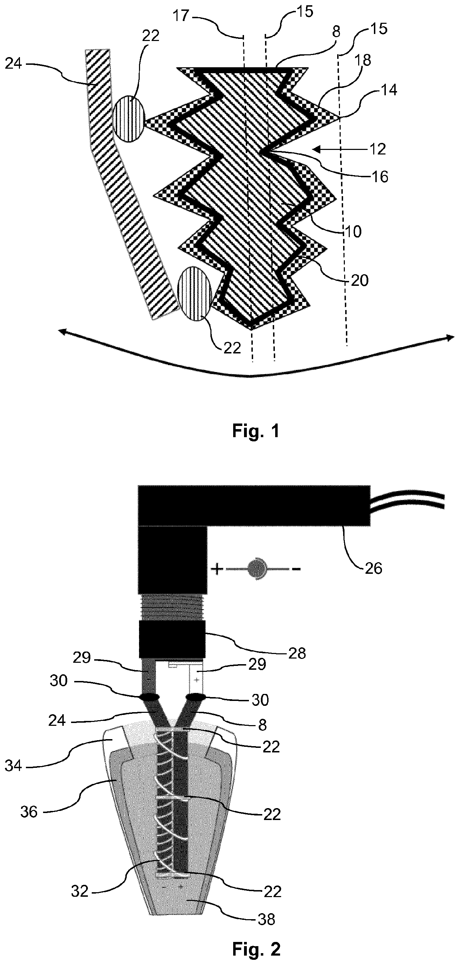

In Fig. 1 ist schematisch ein stark vergrößerter Querschnitt durch die Spitze einer nadelförmigen erfindungsgemäßen Elektrode 8 gezeigt. Die Elektrode besteht unter anderem aus dem Grundmaterial 10, welches Auskerbungen 12 aufweist. Direkt auf dem Grundmaterial 10 wird beim Beschichten mit der Beschichtung 18 aus dotiertem Diamant die Zwischenschicht 20 gebildet. Mit 14 ist beispielhaft ein höchster die beschichtete Auskerbung direkt umgebender Punkt und mit 16 ein tiefster Punkt der beschichteten Auskerbung gekennzeichnet. Zur Messung der Tiefe der beschichteten Auskerbung können Hilfslinien 15 durch den höchsten die beschichtete Auskerbung direkt umgebenden Punkt 14 und den tiefsten Punkt 16 parallel zur Längsachse 17 der Elektrode 8 gezogen werden. Der Abstand zwischen diesen beiden Linien stellt die Tiefe der Auskerbung 12 dar. Der höchste die beschichtete Auskerbung 12 direkt umgebende Punkt 14 kann auch in einem parallel zur Längsachse 17 verlaufenden hier nicht dargestellten Bereich liegen. In diesem Fall gibt es dann eine Mehrzahl gleich hoch gelegener höchster die beschichtete Auskerbung 12 direkt umgebender Punkte 14. Die Summe der maximalen Stärke der Beschichtung 18 und der Zwischenschicht 20 ist geringer als die Tiefe der beschichteten Auskerbung 12. Die Elektrode 8 ist durch ein Verbindungsmittel 22, beispielsweise einen Klebstoff oder einen nicht leitfähigen Diamant mit einer weiteren Elektrode 24 verbunden. Sowohl die weitere Elektrode 24, als auch die Elektrode 8 sind flexibel. Dadurch, dass das Verbindungsmittel 22 die beiden Elektroden 8, 24 nur an wenigen Punkten miteinander verbindet ist durch die Verbindung die Flexibilität der durch die Elektrode 8 und die weitere Elektrode 24 gebildeten Doppelelektrode gewährleistet. Die weitere Elektrode 24 kann beispielweise aus Stahl gebildet sein. Der unterhalb der Elektrode 8 dargestellte Doppelpfeil symbolisiert die Flexibilität der Elektrode.

Fig. 2 zeigt schematisch einen Griff 26 eines zahnärztlichen Instruments, welcher eine Stromverbindung zu einem Sockel 28 bereitstellt. Der Sockel 28 weist elektrische Kontakte 29 auf, an denen die Elektrode 8 und die weitere Elektrode 24 über die Lötstellen 30 durch Löten befestigt worden sind. Im vorliegenden Fall sind sowohl die Elektrode 8 als auch die weitere Elektrode 24 als erfindungsgemäße Elektroden mit einer Beschichtung 18 mit elektrisch leitfähigem Bor-dotierten Diamant ausgestaltet. Die Elektrode 8 ist dabei als Anode geschaltet. Die als Kathode geschaltete weitere Elektrode 24 ist mit einem Teflon-beschichteten Polyethylenfaden 32 aus Polyethylen ultrahohen Molekulargewichts umwickelt. Die Teflonbeschichtung verringert dabei die Reibung auf der Diamantbeschichtung und damit deren mechanische Belastung. Die Umwicklung dient dabei als Abstandshalter. Ein Teil des Polyethylenfadens 32 umwickelt auch die Elektrode 8 und diente zur Fixierung beim Anbringen des Verbindungsmittels 22. Bei dem Verbindungsmittel 22 kann es sich beispielsweise um Cyanoakrylat-Kleber handeln. Die aus der Elektrode 8 und der weiteren Elektrode 24 so gebildete Doppelelektrode wird in den Wurzelkanal 38 eines hier schematisch dargestellten Zahns eingeführt. Sie kann dort unter Strom gesetzt werden und setzt dabei OH-Radikale und andere antimikrobiell wirksamende Spezies frei.

### Herstellung eines Agar-Nährbodens

Flüssiges Standard 1 Nährmedium (St. 1) mit Agar-Agar wird nachfolgend als St. 1 Agar bezeichnet. Die Bestandteile dieses St. 1 Agars sind in nachfolgender Tabelle 1 aufgelistet.

**Tabelle 1: Inhaltsstoffe des Standard 1 Agar-Nährmediums in 400 ml VE-Wasser.**

| **Inhaltsstoffe** | **Menge [g]** | **Hersteller/ Artikelnummer** |
|---|---|---|
| Glukose-Monohydrat | 0,44 | Carl Roth GmbH / 6780.1 |
| Natriumchlorid | 2,34 | Carl Roth GmbH / 3957.1 |
| Agar-Agar | 8,00 | Carl Roth GmbH / 5210.3 |
| Hefeextrakt | 1,20 | Carl Roth GmbH / 2363.3 |
| Pepton aus Casein | 6,00 | Merck KGaA / 1.02239.0500 |

Der pH-Wert wurde mittels Zugabe von Natriumhydroxid auf 7,4 eingestellt. In Petrischalen ausplattiert erstarrte der St. 1 Agar nach wenigen Minuten und diente als Nährboden.

### Inaktivierung von Staphylococcus epidermidis und Bacillus subtilis in einem ausgebohrten Wurzelkanal eines menschlichen Zahns

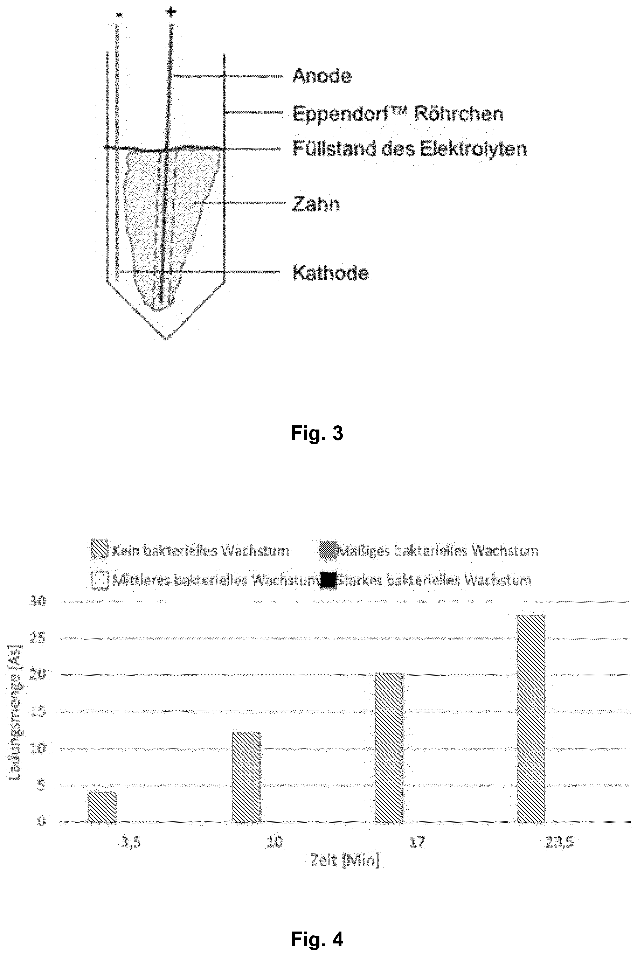

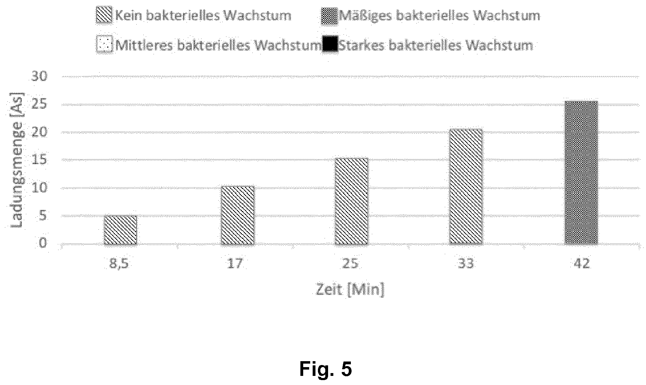

Menschliche extrahierte Zähne mit einem ausgebohrten Wurzelkanal wurden von einem Zahnarzt erhalten. Die Zähne wurden zunächst für mindestens 20 Stunden entweder in einer *Staphylococcus epidermidis* oder *Bacillus subtilis* enthaltenden physiologischen Kochsalzlösung inkubiert. Dabei wurden die Wurzelkanäle mit den jeweiligen Bakterien besiedelt. Anschließend wurden die Zähne mit physiologischer Kochsalzlösung gespült und in physiologischer Kochsalzlösung als Elektrolyt in dem aus Fig. 3 ersichtlichen Versuchsaufbau angeordnet. Die als erfindungsgemäße Elektrode ausgebildete Anode bestand dabei aus einem mit Bor-dotiertem Diamant beschichteten Niobdraht und die Kathode aus Stahl. Es wurde eine Spannung im Bereich von 5 bis 9 Volt angelegt, so dass die in den Figuren 4 und 5 angegebenen Strommengen in der jeweils angegeben Zeit der Behandlung geflossen sind.

Nach der jeweils angegebenen Zeit der Behandlung wurde der jeweilige Zahn gespalten und die dadurch erhaltenen die Oberfläche des Wurzelkanals umfassenden inneren Spaltflächen wurden auf einem St.1 Agar-Nährboden mehrfach eingedrückt und schließlich mit dieser Fläche auf dem Agar gelagert. Der Agar-Nährboden wurde anschließend bei 27 Grad für 1-2 Tage inkubiert. Bakterienwachstum war durch die Bildung von Kolonien zu erkennen. Die Kolonien waren mit dem bloßen Auge als Punkte zu erkennen und wurden subjektiv in kein, mäßiges, mittleres und starkes bakterielles Wachstum kategorisiert.

### Inaktivierung von Staphylococcus epidermidis

Die aus Fig. 4 ersichtlichen Ergebnisse zeigen, dass bereits bei einer Strommenge von 4 As, entsprechend einer Behandlungszeit von 3,5 Minuten, eine vollständige Sterilisation der inneren Wurzelkanaloberfläche erreicht wurde. Auf keiner der Agar-Nährböden zeigte sich ein Bakterienwachstum.

### Inaktivierung von Bacillus subtilis

Die aus Fig. 5 ersichtlichen Ergebnisse zeigen, dass bereits bei einer Strommenge von 5 As, entsprechend einer Behandlungszeit von 8,5 Minuten, eine vollständige Sterilisation der inneren Wurzelkanaloberfläche festgestellt wurde. Ein sich nach einer Behandlungszeit von 42 Minuten und einer Strommenge von 25 As zeigendes mäßiges bakterielles Wachstum kann dadurch verursacht worden sein, dass *Bacillus subtilis* in der Lage ist, Sporen zu bilden und diese Sporen möglicherweise in den Dentinkanälchen der Zahnwurzel enthalten waren. Es wurde weiterhin festgestellt, dass sich bei einer Sporenbildung die zur vollständigen Sterilisation erforderliche Ladungsmenge mehr als verhundertfachen kann.

Die am extrahierten Zahn durchgeführten Versuche zeigen, dass sich eine mit Bor-dotiertem Diamant beschichtete erfindungsgemäße und als Anode geschaltete Elektrode gut zum Desinfizieren eines Wurzelkanals in verhältnismäßig kurzer Zeit eignet. Eine solche Anode kann dazu bei einer Zahnbehandlung in einen er-öffneten Wurzelkanal eingeführt und unter Strom gesetzt werden. Die dazu erforderliche Kathode kann in der Nähe des zu behandelnden Zahns innerhalb der Mundhöhle angeordnet werden. Alternativ kann die erfindungsgemäße Elektrode als Teil einer Doppelelektrode ausgestaltet sein, die als solche in den Wurzelkanal eingeführt werden kann, wie es schematisch in Fig. 2 dargestellt ist.

### Bezugszeichenliste

- 8: Elektrode
- 10: Grundmaterial
- 12: Auskerbung
- 14: höchster die beschichtete Auskerbung direkt umgebender Punkt
- 15: Hilfslinie
- 16: tiefster Punkt
- 17: Längsachse
- 18: Beschichtung
- 20: Zwischenschicht
- 22: Verbindungsmittel
- 24: weitere Elektrode
- 26: Griff
- 28: Sockel
- 29: elektrischer Kontakt
- 30: Lötstelle
- 32: Polyethylenfaden
- 34: Zahnschmelz
- 36: Dentin
- 38: Wurzelkanal

## Patentansprüche

1. Flexible Elektrode (8) aus einem metallischen Grundmaterial (10) mit einer Beschichtung (18) aus polykristallinem dotiertem elektrisch leitfähigem Diamant und einer Zwischenschicht (20) zwischen dem Grundmaterial (10) und der Beschichtung (18), wobei das Grundmaterial (10) nadelförmig, drahtförmig oder bandförmig ausgebildet ist, wobei in dem Grundmaterial (10) um eine Längsachse (17) des Grundmaterials (10) herum mindestens eine umlaufende Auskerbung (12) enthalten ist.

2. Flexible Elektrode (8) nach Anspruch 1, wobei die Auskerbung (12) mit der Zwischenschicht (20) und der Beschichtung (18) tiefer ist als die Summe der maximalen Stärke der Beschichtung (18) und der maximalen Stärke der Zwischenschicht (20).

3. Flexible Elektrode (8) nach Anspruch 1 oder 2, wobei der Diamant mit Bor oder Phosphor dotiert ist.

4. Flexible Elektrode (8) nach einem der vorherigen Ansprüche, wobei das Grundmaterial (10) aus Titan, Niob, Tantal, Eisen oder einer diese Metalle enthaltenden Legierung oder einem Stahl besteht.

5. Flexible Elektrode (8) nach einem der vorherigen Ansprüche, wobei die Zwischenschicht (20) aus einem Metallkarbid, einem Metallnitrid, einem Metallborid oder einer mindestens zwei der genannten Karbide enthaltenden Mischverbindung besteht.

6. Flexible Elektrode (8) nach Anspruch 4, wobei die Zwischenschicht (20) eine Schichtdicke von höchstens 10 µm, insbesondere höchstens 5 µm, und mindestens 50 nm aufweist.

7. Flexible Elektrode (8) nach einem der vorherigen Ansprüche, wobei die Beschichtung (18) mit Diamant eine Schichtdicke von höchstens 3 µm, insbesondere weniger als 2 µm, und von mindestens 100 nm aufweist.

8. Flexible Elektrode (8) nach einem der vorherigen Ansprüche zur Verwendung bei einer antimikrobiellen Behandlung eines mikrobiell infizierten Endodonts oder Parodonts eines Zahnes oder eines mikrobiell infizierten periimplantären Gewebes eines Säugetiers oder Menschen.

9. Flexible Elektrode (8) nach Anspruch 8, wobei die Elektrode (8) mit einer weiteren Elektrode (24) eine Doppelelektrode bildet, wobei die weitere Elektrode (24) ebenfalls nadelförmig, drahtförmig oder bandförmig ausgebildet ist, wobei die Elektrode (8) und die weitere Elektrode (24) in einem Bereich durch mindestens ein Verbindungsmittel (22) oder ein Verbindungsmittel (22) und ein Abstandshaltemittel so miteinander verbunden sind, dass dadurch ein elektrischer Kontakt zwischen der Elektrode (8) und der weiteren Elektrode (24) ausgeschlossen ist und in mindestens 80 % der Länge dieses Bereichs zwischen der Elektrode (8) und der weiteren Elektrode (24) ein freier, bei der Behandlung von einem Elektrolyten einzunehmender Raum gewährleistet ist, wobei ein Abstand zwischen der Elektrode (8) und der weiteren Elektrode (24) innerhalb dieses Raums zwischen 40 µm und 300 µm beträgt.

10. Flexible Elektrode (8) nach Anspruch 9, wobei bei der Behandlung die Elektrode (8) als Anode und die weitere Elektrode (24) als Kathode geschaltet ist.

11. Flexible Elektrode (8) nach Anspruch 9 oder 10, wobei die weitere Elektrode (24) aus demselben Grundmaterial (10) wie die Elektrode (8) besteht und wie die Elektrode (8) aufgebaut ist.

12. Flexible Elektrode (8) nach Anspruch 11, wobei bei der Behandlung die Elektrode (8) und die weitere Elektrode (24) jeweils abwechselnd als Anode und die jeweils andere der beiden Elektroden (8, 24) als Kathode geschaltet ist.

13. Flexible Elektrode (8) nach einem der Ansprüche 9 bis 12, wobei der Abstand zwischen der Elektrode (8) und der weiteren Elektrode (24) innerhalb des Raums zwischen 40 µm und 200 µm, insbesondere zwischen 40 µm und 110 µm, beträgt.

14. Flexible Elektrode (8) nach einem der Ansprüche 9 bis 13, wobei das Verbindungsmittel (22) ein elektrisch isolierender Klebstoff, insbesondere ein Epoxidharz, ein Kunststofffaden oder ein nicht leitender Diamant und das Abstandshaltemittel Teflon oder ein elektrisch isolierend beschichteter Draht ist.

15. Flexible Elektrode (8) nach einem der Ansprüche 8 bis 14, wobei die Behandlung des mikrobiell infizierten Endodonts nach der antimikrobiellen Behandlung ein zumindest teilweises Verfüllen eines im Zahn bestehenden oder durch eine weitere Behandlung gebildeten Hohlraums mit einem durch elektrische Aktivierung im Hohlraum aushärtenden Kunststoff oder mit einem anorganischen Stoff, dessen Präzipitation aus einer Lösung im Hohlraum durch anodische Oxidation initiiert wird, umfasst, wobei die Aktivierung oder die anodische Oxidation mittels der Elektrode im Hohlraum erfolgt.

## Claims

1. Flexible electrode (8) made of a metallic base material (10) with a coating (18) of polycrystalline doped electrically conductive diamond and an intermediate layer (20) between the base material (10) and the coating (18), wherein the base material (10) is of needle-shaped, wire-shaped or band-shaped form, wherein at least one circumferential groove (12) is contained in the base material (10) around a longitudinal axis (17) of the base material (10).

2. Flexible electrode (8) according to claim 1, wherein the groove (12) with the intermediate layer (20) and the coating (18) is deeper than the sum of the maximum thickness of the coating (18) and the maximum thickness of the intermediate layer (20).

3. Flexible electrode (8) according to claim 1 or 2, wherein the diamond is doped with boron or phosphorus.

4. Flexible electrode (8) according to any one of the preceding claims, wherein the base material (10) consists of titanium, niobium, tantalum, iron or an alloy containing these metals, or a steel.

5. Flexible electrode (8) according to any one of the preceding claims, wherein the intermediate layer (20) consists of a metal carbide, a metal nitride, a metal boride or a mixed compound containing at least two of said carbides.

6. Flexible electrode (8) according to claim 4, wherein the intermediate layer (20) has a layer thickness of at most 10 µm, in particular at most 5 µm, and at least 50 nm.

7. Flexible electrode (8) according to any one of the preceding claims, wherein the coating (18) with diamond has a layer thickness of at most 3 µm, in particular less than 2 µm, and of at least 100 nm.

8. Flexible electrode (8) according to any one of the preceding claims for use in an antimicrobial treatment of a microbially infected endodont or periodont of a tooth or a microbially infected peri-implant tissue of a mammal or human.

9. Flexible electrode (8) according to claim 8, wherein the electrode (8) forms a double electrode with a further electrode (24), wherein the further electrode (24) is likewise of needle-shaped, wire-shaped or band-shaped form, wherein the electrode (8) and the further electrode (24) are connected to one another in an area by at least one connecting means (22) or a connecting means (22) and a spacing means in such a way, that thereby an electrical contact between the electrode (8) and the further electrode (24) is excluded and in at least 80% of the length of this area between the electrode (8) and the further electrode (24) a free space to be occupied by an electrolyte during the treatment is ensured, wherein a distance between the electrode (8) and the further electrode (24) within this space is between 40 µm and 300 µm.

10. Flexible electrode (8) according to claim 9, wherein the electrode (8) is connected as anode and the further electrode (24) is connected as cathode during the treatment.

11. Flexible electrode (8) according to claim 9 or 10, wherein the further electrode (24) consists of the same base material (10) as the electrode (8) and is constructed like the electrode (8).

12. Flexible electrode (8) according to claim 11, wherein in the treatment the electrode (8) and the further electrode (24) are each alternately connected as anode and the other of the two electrodes (8, 24) is connected as cathode.

13. Flexible electrode (8) according to any one of claims 9 to 12, wherein the distance between the electrode (8) and the further electrode (24) within the space is between 40 µm and 200 µm, in particular between 40 µm and 110 µm.

14. Flexible electrode (8) according to any one of claims 9 to 13, wherein the connecting means (22) is an electrically insulating adhesive, in particular an epoxy resin, a plastic thread or a non-conductive diamond, and the spacing means is Teflon or an electrically insulating coated wire.

15. Flexible electrode (8) according to any one of claims 8 to 14, wherein the treatment of the microbially infected endodont after the antimicrobial treatment comprises at least partially filling a cavity existing in the tooth or formed by a further treatment with a resin hardening by electrical activation in the cavity or with an inorganic substance whose precipitation from a solution in the cavity is initiated by anodic oxidation, wherein the activation or the anodic oxidation takes place by means of the electrode in the cavity.

## Revendications

1. Electrode flexible (8) en un matériau de base métallique (10) avec un revêtement (18) en diamant polycristallin dopé électriquement conducteur et une couche intermédiaire (20) entre le matériau de base (10) et le revêtement (18), dans laquelle le matériau de base (10) est réalisé en forme d'aiguille, en forme de fil métallique ou en forme de bande, dans laquelle au moins une encoche périphérique (12) est contenue dans le matériau de base (10) autour d'un axe longitudinal (17) du matériau de base (10).

2. Electrode flexible (8) selon la revendication 1, dans laquelle l'encoche (12) avec la couche intermédiaire (20) et le revêtement (18) est plus profonde que la somme de l'épaisseur maximale du revêtement (18) et de l'épaisseur maximale de la couche intermédiaire (20).

3. Electrode flexible (8) selon la revendication 1 ou 2, dans laquelle le diamant est dopé avec du bore ou du phosphore.

4. Electrode flexible (8) selon une des revendications précédentes, dans laquelle le matériau de base (10) se compose de titane, niobium, tantale, fer ou d'un alliage contenant ces métaux ou d'un acier.

5. Electrode flexible (8) selon une des revendications précédentes, dans laquelle la couche intermédiaire (20) se compose d'un carbure métallique, d'un nitrure métallique, d'un borure métallique ou d'un composé mixte contenant au moins deux des carbures cités.

6. Electrode flexible (8) selon la revendication 4, dans laquelle la couche intermédiaire (20) présente une épaisseur de couche d'au plus 10 µm, notamment d'au plus 5 µm, et d'au moins 50 nm.

7. Electrode flexible (8) selon une des revendications précédentes, dans laquelle le revêtement (18) avec du diamant présente une épaisseur de couche d'au plus 3 µm, notamment de moins de 2 µm, et d'au moins 100 nm.

8. Electrode flexible (8) selon une des revendications précédentes pour l'utilisation lors d'un traitement antimicrobien d'un endodonte ou parodonte à infection microbienne d'une dent ou d'un tissu péri-implantaire à infection microbienne d'un mammifère ou être humain.

9. Electrode flexible (8) selon la revendication 8, dans laquelle l'électrode (8) forme avec une autre électrode (24) une double électrode, dans laquelle l'autre électrode (24) est également réalisée en forme d'aiguille, en forme de fil métallique ou en forme de bande, dans laquelle l'électrode (8) et l'autre électrode (24) sont connectées l'une à l'autre dans une région par au moins un moyen de connexion (22) ou un moyen de connexion (22) et un moyen d'écartement de sorte qu'un contact électrique est de ce fait exclu entre l'électrode (8) et l'autre électrode (24), et un espace libre, à occuper lors du traitement par un électrolyte est garanti dans au moins 80 % de la longueur de cette région entre l'électrode (8) et l'autre électrode (24), dans laquelle un écart entre l'électrode (8) et l'autre électrode (24) au sein de cet espace est compris entre 40 µm et 300 µm.

10. Electrode flexible (8) selon la revendication 9, dans laquelle l'électrode (8) est montée en tant qu'anode et l'autre électrode (24) est montée en tant que cathode lors du traitement.

11. Electrode flexible (8) selon la revendication 9 ou 10, dans laquelle l'autre électrode (24) se compose du même matériau de base (10) que l'électrode (8) et est constituée comme l'électrode (8).

12. Electrode flexible (8) selon la revendication 11, dans laquelle l'électrode (8) et l'autre électrode (24) sont chacune montées en alternance en tant qu'anode et l'autre électrode respective des deux électrodes (8, 24) est montée en tant que cathode lors du traitement.

13. Electrode flexible (8) selon une des revendications 9 à 12, dans laquelle l'écart entre l'électrode (8) et l'autre électrode (24) au sein de l'espace est compris entre 40 µm et 200 µm, notamment entre 40 µm et 110 µm.

14. Electrode flexible (8) selon une des revendications 9 à 13, dans laquelle le moyen de connexion (22) est un adhésif électriquement isolant, notamment une résine époxyde, un fil plastique ou un diamant non conducteur et le moyen d'écartement est du Téflon ou un fil métallique revêtu électriquement isolant.

15. Electrode flexible (8) selon une des revendications 8 à 14, dans laquelle le traitement de l'endodonte à infection microbienne après le traitement antimicrobien comprend une obturation au moins partielle d'un espace creux existant dans la dent ou formé par un autre traitement avec une matière synthétique durcissant par activation électrique dans l'espace creux ou avec une substance inorganique dont la précipitation est initiée à partir d'une solution dans l'espace creux par oxydation anodique, dans laquelle l'activation ou l'oxydation anodique s'effectue au moyen de l'électrode dans l'espace creux.
